# EUROPÄISCHE PATENTSCHRIFT

(11) **EP 2 072 517 B1**
(45) Veröffentlichungstag und Bekanntmachung des Hinweises auf die Patenterteilung: **21.01.2015**
(21) Anmeldenummer: 07400033.2
(22) Anmeldetag: 21.12.2007
(51) Int. Cl.: C07D 471/04, C09K 11/06, H05B 33/14, H05B 33/20, H01L 51/00, H01L 51/50

(54) **Asymmetrische Phenanthroline, deren Herstellungsverfahren und diese enthaltendes dotiertes organisches Halbleitermaterial**
Asymmetric phenanthrolins, method for their manufacture and doped organic semiconductor material containing them
Phénanthrolines asymétriques, procédé de fabrication et matériau semi-conducteur organique dopé ainsi obtenu

(43) Veröffentlichungstag der Anmeldung: 24.06.2009
(73) Patentinhaber: Novaled GmbH, 01307 Dresden (DE)
(72) Erfinder: Kussler, Manfred, Dr., 79774 Albbruck (DE); Lux, Andrea, Dr., 07768 Kahla (DE); Salbeck, Josef, Dr., 34260 Kaufungen (DE)
(74) Vertreter: Bittner, Thomas L.

(56) Entgegenhaltungen:
- EP-A- 1 786 050
- WO-A-2004/026870
- US-A1- 2006 138 453

## Beschreibung

Die vorliegende Erfindung betrifft asymmetrisch substituierte 1,10-Phenanthroline, Verfahren zu deren Herstellung sowie dotierte organische Halbleitermaterialien, in denen diese eingesetzt werden.

Seit der Demonstration von organischen Leuchtdioden und Solarzellen 1987 [C.W. Tang et al., Appl. Phys. Lett. 51 (12), 913 (1987)] sind aus organischen Dünnschichten aufgebaute Bauelemente Gegenstand intensiver Forschung. Derartige Schichten besitzen vorteilhafte Eigenschaften für die genannten Anwendungen, wie z.B. effiziente Elektrolumineszenz für organische Leuchtdioden, hohe Absorptionskoeffizienten im Bereich des sichtbaren Lichtes für organische Solarzellen, preisgünstige Herstellung der Materialien und Fertigung der Bauelemente für einfachste elektronische Schaltungen, u.a. Kommerzielle Bedeutung hat bereits der Einsatz organischer Leuchtdioden für Displayanwendungen.

Die Leistungsmerkmale (opto-)elektronischer mehrschichtiger Bauelemente werden unter anderem von der Fähigkeit der Schichten zum Transport der Ladungsträger bestimmt. Im Falle von Leuchtdioden hängen die ohmschen Verluste in den Ladungstransportschichten beim Betrieb mit der Leitfähigkeit zusammen, was einerseits direkten Einfluss auf die benötigte Betriebsspannung hat, andererseits aber auch die thermische Belastung des Bauelements bestimmt. Weiterhin kommt es in Abhängigkeit von der Ladungsträgerkonzentration der organischen Schichten zu einer Bandverbiegung in der Nähe eines Metallkontakts, die die Injektion von Ladungsträgern erleichtert und damit den Kontaktwiderstand verringern kann. Ähnliche Überlegungen führen auch für organische Solarzellen zu dem Schluss, dass deren Effizienz auch durch die Transporteigenschaften für Ladungsträger bestimmt wird.

Durch Dotierung von Löchertransportschichten mit einem geeigneten Akzeptormaterial (p-Dotierung) bzw. von Elektronentransportschichten mit einem Donatormaterial (n-Dotierung) kann die Ladungsträgerdichte in organischen Festkörpern (und damit die Leitfähigkeit) beträchtlich erhöht werden. Darüber hinaus sind in Analogie zur Erfahrung mit anorganischen Halbleitern Anwendungen zu erwarten, die gerade auf Verwendung von p- und n-dotierten Schichten in einem Bauelement beruhen und anders nicht denkbar wären. In US 5,093,698 ist die Verwendung von dotierten Ladungsträgertransportschichten (p-Dotierung der Löchertransportschicht durch Beimischung von akzeptorartigen Molekülen, n-Dotierung der Elektronentransportschicht durch Beimischung von donatorartigen Molekülen) in organischen Leuchtdioden beschrieben.

Folgende Ansätze sind bisher für die Verbesserung der Leitfähigkeit von organischen aufgedampften Schichten bekannt:
1. Erhöhung der Ladungsträgerbeweglichkeit durch
   a) Verwendung von Elektronentransportschichten bestehend aus organischen Radikalen (US 5,811,833),
   b) Erzeugung hoch geordneter Schichten, die eine optimale Überlappung der π-Orbitale der Moleküle erlauben,
2. Erhöhung der Dichte der beweglichen Ladungsträger durch
   a) Reinigung und schonende Behandlung der Materialien, um die Ausbildung von Ladungsträgerhaftstellen zu vermeiden,
   b) Dotierung organischer Schichten mittels
      aa) anorganischer Materialien (Alkalimetalle: J. Kido et al., US 6,013,384; J. Kido et al., Appl. Phys. Lett. 73, 2866 (1998), Oxidationsmittel wie Iod, SbCl₅ etc.)
      bb) organischer Materialien (TNCQ: M. Maitrot et al., J. Appl. Phys., 60 (7), 2396-2400 (1986), F4TCNQ: M. Pfeiffer et al., Appl. Phys. Lett., 73 (22), 3202 (1998), BEDT-TTF: A. Nollau et al., J. Appl. Phys., 87 (9), 4340 (2000), Naphthalendicarbonsäureamide: M.Thomson et al., WO03088271, kationische Farbstoffe: A.G. Werner, Appl. Phys. Lett. 82,4495 (2003))
      cc) Organometallverbindungen (Metallocene: M.Thomson et al., WO03088271)
      dd) Metallkomplexe (Ru⁰(terpy)₃: K. Harada et al., Phys. Rev. Lett. 94, 036601 (2005)

Während es für die p-Dotierung bereits ausreichend starke, organische Dotanden gibt (F4TCNQ), stehen für die n-Dotierung nur anorganische Materialien, z.B. Cäsium, zur Verfügung. Durch deren Einsatz konnte auch bereits eine Verbesserung der Leistungsparameter von OLEDs erzielt werden. So erzielt man durch Dotierung der Löchertransportschicht mit dem Akzeptormaterial F4TCNQ eine drastische Reduzierung der Betriebsspannung der Leuchtdiode (X. Zhou et al., Appl. Phys. Lett., 78 (4), 410 (2001).). Ein ähnlicher Erfolg ist durch die Dotierung der elektronen-transportierenden Schicht mit Cs oder Li zu erzielen (J. Kido et al., Appl. Phys. Lett., 73 (20), 2866 (1998); J.-S. Huang et al, Appl. Phys. Lett., 80, 139 (2002)).

Ein großes Problem bei der n-Dotierung war lange Zeit, dass für diese nur anorganische Materialien zur Verfügung standen. Die Verwendung von anorganischen Materialien hat jedoch den Nachteil, dass die verwendeten Atome bzw. Moleküle aufgrund ihrer geringen Größe leicht im Bauelement diffundieren können und somit eine definierte Herstellung z.B. scharfer Übergänge von p- dotierten zu n-dotierten Gebieten erschweren.

Die Diffusion sollte demgegenüber bei Verwendung großer, Raum ausfüllender, organischer Moleküle als Dotanden eine untergeordnete Rolle spielen, da Platzwechselvorgänge nur unter Überwindung höherer Energiebarrieren möglich sind.

Es ist seit vielen Jahren insbesondere bei organischen polymeren Halbleitermaterialien bekannt, daß ein wirksamer Elektronentransfer von einem Dotanden (beispielsweise Natrium) auf die organische Matrix (beispielsweise Polyacetylen) nur möglich ist, wenn die Differenz zwischen HOMO-Energieniveau (= Ionisationspotential) des Dotanden und dem LUMO-Energieniveau (= Elektronenaffinität) der Matrix möglichst gering ist.

Zur Bestimmung des Ionsiationspotentials ist Ultraviolett-Photoelektronenspektroskopie (UPS) die bevorzugte Methode (z.B. R. Schlaf et al., J. Phys. Chem. B 103, 2984 (1999)). Eine verwandte Methode, inverse Photoelektronenspektroskopie (IPES), wird zur Bestimmung von Elektronenaffinitäten herangezogen (zB. W. Gao et. al, Appl. Phys. Lett. 82, 4815 (2003), ist jedoch weniger etabliert. Alternativ können die Festkörperpotentiale durch elektrochemische Messungen von Oxidationspotentialen Eₒₓ bzw. Reduktionspotentialen E_{red} in der Lösung, z.B. durch Cyclovoltammetrie (engl. Cyclic Voltammetry, CV), abgeschätzt werden (z.B. J.D. Anderson, J. Amer. Chem. Soc. 120, 9646 (1998)). Mehrere Arbeiten geben empirische Formeln zur Umrechnung der elektrochemischen Spannungsskala (Oxidationspotentiale) in die physikalische (absolute) Energieskala (Ionisationspotentiale) an, z.B. B.W. Andrade et al., Org. Electron. 6, 11 (2005); T.B. Tang, J. Appl. Phys. 59, 5 (1986); V.D. Parker, J. Amer. Chem. Soc. 96, 5656 (1974); L.L. Miller, J. Org. Chem. 37, 916 (1972), Y. Fu et al., J. Amer. Chem. Soc. 127, 7227 (2005). Zwischen Reduktionspotential und Elektronenaffinität ist keine Korrelation bekannt, da sich Elektronenaffinitäten nur schwer messen lassen. Deshalb werden vereinfacht die elektrochemische und die physikalische Energieskala über IP=4.8 eV+ e*Eₒₓ (vs. Ferrocen/Ferrocenium) bzw. EA=4.8eV +e*E_{red} (vs. Ferrocen/Ferrocenium) ineinander umgerechnet, wie in B.W. Andrade, Org. Electron. 6, 11 (2005) (siehe dort auch Ref. 25-28) beschrieben. Die Umrechnung von verschiedenen Standardpotentialen bzw. Redoxpaaren wird beispielsweise in A.J. Bard, L.R. Faulkner, "Electrochemical Methods: Fundamentals and Applications", Wiley, 2. Auflage 2000 beschrieben.
Aus der obigen Darstellung ergibt sich somit, daß die genaue Ermittlung sämtlicher Energiewerte gegenwärtig nicht möglich ist und die dargestellten Werte lediglich als Richtgrößen aufgefaßt werden können.

Beim n-Dotieren fungiert der Dotand als Elektronendonator und überträgt Elektronen auf eine Matrix, welche sich durch eine genügend hohe Elektronenaffinität auszeichnet. Das heißt die Matrix wird reduziert. Durch den Transfer von Elektronen vom n-Dotanden auf die Matrix wird die Ladungsträgerdichte der Schicht erhöht. Inwieweit ein n-Dotand in der Lage ist, Elektronen an eine geeignete, elektronenaffine Matrix abzugeben und dadurch die Ladungsträgerdichte und damit einhergehend die elektrische Leitfähigkeit zu erhöhen, hängt wiederum von der relativen Lage des HOMOs des n-Dotanden sowie des LUMOs der Matrix zueinander ab. Wenn das HOMO des n-Dotanden über dem LUMO der elektronenaffinen Matrix liegt, kann ein Elektronentransfer stattfinden. Wenn das HOMO des n-Dotanden unter dem LUMO der elektronenaffinen Matrix liegt, kann ebenfalls ein Elektronentransfer stattfinden, vorausgesetzt, dass die Energiedifferenz zwischen den beiden Orbitalen ausreichend niedrig ist, um eine gewisse thermische Population des höheren Energieorbitals zu ermöglichen. Je kleiner diese Energiedifferenz ist, desto höher sollte die Leitfähigkeit der resultierenden Schicht sein. Die höchste Leitfähigkeit ist jedoch zu erwarten für den Fall, dass das HOMO-Niveau des n-Dotanden über dem LUMO-Niveau der elektronenaffinen Matrix liegt. Die Leitfähigkeit ist praktisch messbar und ein Maß dafür, wie gut der Elektronenübertrag vom Donor auf den Akzeptor funktioniert, vorausgesetzt, dass die Ladungsträgermobilitäten verschiedener Matrizen vergleichbar sind.

Die Leitfähigkeit einer Dünnschichtprobe wird mit der 2-Punkt-Methode gemessen. Dabei werden auf ein Substrat Kontakte aus einem leitfähigen Material aufgebracht, z.B. Gold oder Indium-Zinn-Oxid. Danach wird die zu untersuchende Dünnschicht großflächig auf das Substrat aufgebracht, so dass die Kontakte von der Dünnschicht überdeckt werden. Nach Anlegen einer Spannung an die Kontakte wird der dann fließende Strom gemessen. Aus der Geometrie der Kontakte und der Schichtdicke der Probe ergibt sich aus dem so bestimmten Widerstand die Leitfähigkeit des Dünnschichtmaterials. Die 2-Punkt-Methode ist anwendbar, wenn der Widerstand der Dünnschicht wesentlich größer ist als der Widerstand der Zuleitungen oder der Kontaktwiderstand. Experimentell wird dies durch einen genügend hohen Kontaktabstand gewährleistet, somit kann die Linearität der Strom-Spannungs-Kennlinie überprüft werden.

Untersuchungen der Erfinder haben gezeigt, daß Metallkomplexdotanden der Struktur I 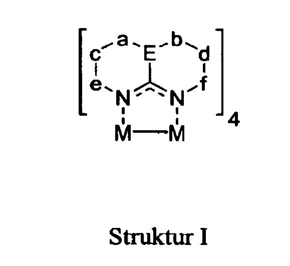 vorteilhaft als Dotanden für ein organisches Matrixmaterial eingesetzt werden können, da ein solcher Dotand das oben beschriebene Diffusionsproblem löst. Die Definitionen für a-f, E und M sind wie in Anspruch 10 beschrieben. Aus diesem Grunde wurde ein Dotand mit der Struktur Ia 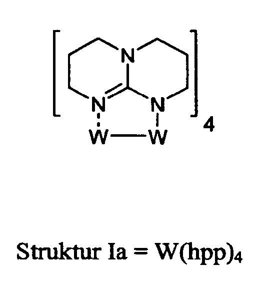 als Dotand für herkömmliche Elektronentransportmaterialien, wie Alq₃ (Tris(8-hydroxyquinolinato)-aluminium) oder BPhen (4,7-Diphenyl-1,10-phenanthrolin), getestet.

Das Gasphasenionisationspotential des Dotanden mit der Struktur Ia beträgt 3,6 eV. Das entsprechende Ionisationspotential des Festkörpers kann nach Y. Fu et al. (J. Am. Chem. Soc. 2005, 127, 7227-7234) abgeschätzt werden und beträgt etwa 2,5 eV.

Die Ergebnisse sind in der folgenden Tabelle 1 dargestellt.

**Tabelle 1: CV-Daten, empirisch ermittelte LUMO-Energien und gemessene Leitfähigkeiten verschiedener Elektronentransportmaterialien (BAlq₂ = Bis(2-methyl-8-quinolinato)-4-(phenylphenolato)aluminium-(III), BPhen = Bathophenanthrolin, Alq3: (Tris(8-hydroxyquinoline-)aluminium)**

| Matrixmaterial | LUMO in eV (ermittelt über CV mit Fc/Fc⁺ als internen Standard) | σ. (Leitfähigkeit) in S/cm undotiert | σ. (Leitfähigkeit) in S/cm bei einer Dotierkonzentration von 5 mol% |
|---|---|---|---|
| Alq₃ | 2,4 | < 1E-10 | 9,2E-8 |
| BPhen | 2,42 | < 1E-10 | 4E-9 |
| BAlq₂ | 2,39 | < 1E-10 | 8e-8 |

Wie aus der Tabelle 1 entnommen werden kann, sind die erzielten Leitfähigkeiten mit den bekannten Matrixmaterialien noch unzureichend und sehr gering.

EP 1 786 050 offenbart Phenanthrolinderivate, die sowohl symmetrisch als auch asymmetrisch substituiert sein können zur Verwendung in Halbleitermaterialien.

Aufgabe der vorliegenden Erfindung ist es, verbesserte Matrixmaterialien für dotierte organische Halbleitermaterialien bereitzustellen, die die Nachteile des Stands der Technik überwinden. Insbesondere sollen die Leitfähigkeiten durch Variation der Matrixmaterialien verbessert werden. Insbesondere soll ein Halbleitermaterial zur Verfügung gestellt werden, das eine erhöhte Ladungsträgerdichte und effektive Ladungsträgerbeweglichkeit sowie eine verbesserte Leitfähigkeit aufweist. Das Halbleitermaterial soll eine hohe thermische Stabilität zeigen, die sich beispielsweise aus höheren Glasübergangspunkten, höheren Sublimationstemperaturen und höheren Zersetzungstemperaturen ergibt.

Schließlich ist es eine Aufgabe der Erfindung, ein Herstellungsverfahren für die Matrixmaterialien bereitzustellen.

Die Aufgabe der Erfindung wird gelöst durch asymmetrisch substituierte Phenanthroline nach Anspruch 1.

Diese asymmetrisch substituierten Phenanthroline können als Matrixmaterialien in dotierten organischen Halbleitermaterialien eingesetzt werden und führen dann zu verbesserten Leitfähigkeitsergebnissen.

Die erfindungsgemäßen Phenanthroline zeigen insbesondere vielversprechende Eigenschaften beim Einsatz als Elektronentransportschicht in organischen lichtemittierenden Dioden. Die hier beanspruchten Phenanthroline sind bislang nicht durch herkömmliche Verfahren darstellbar gewesen. Sie weisen insbesondere auch den Vorteil auf, die Kristallisationsneigung zu unterbinden und somit eine morphologische stabile Schicht nach Verdampfung bereitzustellen, was für organische lichtemittierende Dioden von besonderer Bedeutung ist.

Die asymmetrisch substituierten Phenanthroline können gemäß einem Verfahren hergestellt werden, das die folgenden Schritte umfaßt:
(ia) Umsetzung eines substituierten Methylenhydrochinolinons **5** mit einem Enamin unter basischen Bedingungen zur Darstellung eines 1,4,5,6-Tetrahydrophenanthrolins **6** und anschließende Oxidation zur Darstellung eines 5,6-Dihydrophenanthrolins **7** gemäß dem folgenden Reaktionsschema: 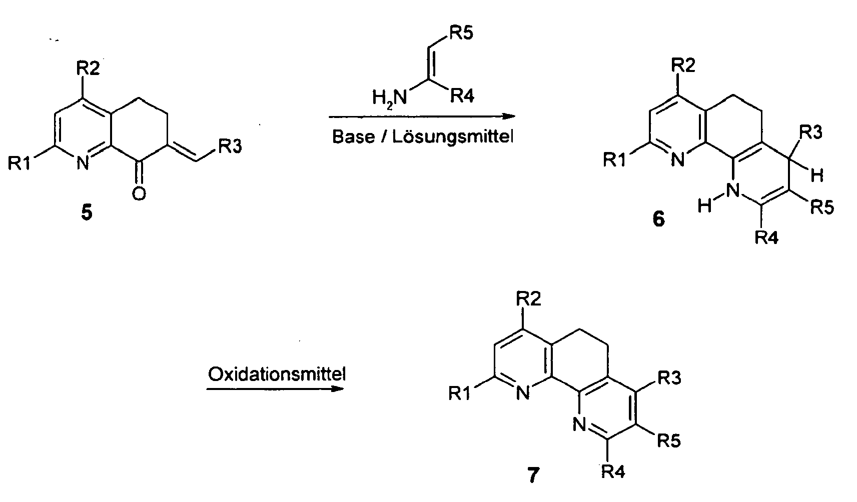
(iia) Aromatisierung des 5,6-Dihydrophenanthrolins **7** zum asymmetrisch substituierten Phenanthrolin **8** gemäß dem folgenden Reaktionsschema: 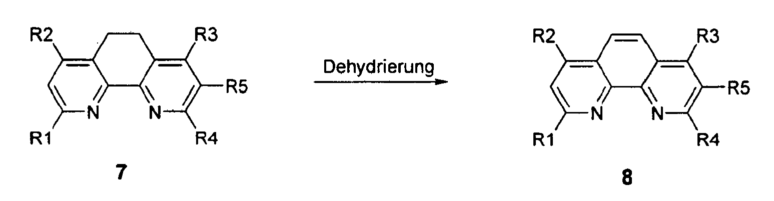 oder
(ib) Umsetzung eines substituierten Methylenhydrochinolinons **5** mit einem Enamin unter basischen Bedingungen zur Darstellung eines 1,4,5,6-Tetrahydrophenanthrolins **6** und anschließende direkte Oxidation zum asymmetrisch substituierten Phenanthrolin **8,**
wobei R¹, R², R³, R⁴ und R⁵ wie oben in Anspruch 1 definiert sind.

Auch ist bevorzugt vorgesehen, daß zur Oxidation des 1,4,5,6-Tetrahydrophenanthrolins **6** in Schritt (i) Methansulfonsäure oder Chloranil eingesetzt wird.

Erfindungsgemäß wurde festgestellt, dass es zwei Verfahren zur Herstellung der asymmetrisch substituierten Phenanthroline gibt. In einem ersten Verfahren erfolgt zunächst die Umsetzung eines substituierten Methylenhydrochinolinons **5** mit Enamin unter basischen Bedingungen zur Darstellung eines 1,4,5,6-Tetrahydrophenanthrolins **6** und eine anschließende Oxidation zur Darstellung eines 5,6-Dihydrophenanthrolins **7,** welches dann anschließend zum asymmetrisch substituierten Phenanthrolin **8** aromatisiert wird.

Der Schritt zur Herstellung des 5,6-Dihydrophenanthrolins **7** in einem alternativen Verfahrenkann eingespart werden, so dass dann eine direkte Oxidation des 1,4,5,6-Tetrahydrophenanthrolins **6** zum asymmetrisch substituierten Phenanthrolin **8** erfolgt. Der Syntheseweg hängt dabei im Wesentlichen von dem eingesetzten Oxidationsmittel ab. Wird beispielsweise Methansulfonsäure als Oxidationsmittel eingesetzt, wird das 5,6-Dihydrophenanthrolin **7** erhalten, während bei Verwendung von Chloranil direkt das asymmetrisch substituierte Phenanthrolin **8** erhalten wird. Dies wird aus den unten beschriebenen Beispielen offensichtlich. Erfindungsgemäß wurde festgestellt, dass bei einer direkten Oxidation beispielsweise mit Chloranil weniger Nebenprodukte erzeugt werden sowie ein Syntheseschritt eingespart werden kann, was in beträchtlichen Vorteilen resultiert.

Ebenfalls wird bevorzugt vorgeschlagen, daß die Aromatisierung in Schritt (ii) durch Pd-katalysierte Dehydrierung mittels Pd/C erfolgt.

Erfindungsgemäß ist auch ein dotiertes organisches Halbleitermaterial umfassend mindestens ein organisches Matrixmaterial, das mit mindestens einem Dotanden dotiert ist, wobei das Matrixmaterial ein erfindungsgemäßes, asymmetrisch substituiertes Phenanthrolin ist.

Dabei ist bevorzugt, daß das Matrixmaterial reversibel reduzierbar ist.

Alternativ wird vorgeschlagen, daß das Matrixmaterial bei einer Reduktion durch den Dotanden in stabile, redox-inaktive Bestandteile zerfällt.

Der Dotand kann ein Metallkomplex sein.

Bevorzugt weist der Metallkomplex eine Struktur **I** auf, die in der Patentanmeldung DE102004010954 offenbart ist: 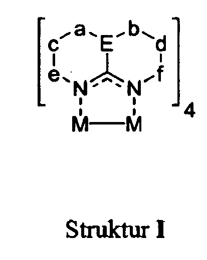 wobei M ein Übergangsmetall ist, vorzugsweise Mo oder W; und wobei
- die Strukturelemente a-f die Bedeutung: a= -CR₉R₁₀-, b = -CR₁₁R₁₂-, c = -CR₁₃R₁₄-, d = R₁₅R₁₆-, e = -CR₁₇R₁₈- und f = -CR₁₉R₂₀- aufweisen können, wobei R₉-R₂₀ unabhängig Wasserstoff, Alkyl mit C₁-C₂₀, Cycloalkyl mit C₃-C₂₀, Alkenyl mit C₁-C₂₀, Alkinyl mit C₂-C₂₀, Aryl, Heteroaryl, -NR²¹R²¹ oder -OR²¹ sind, wobei R²¹ = Alkyl mit C₁-C₂₀, Cycloalkyl mit C₃-C₂₀, Alkenyl mit C₂-C₂₀, Alkinyl mit C₂-C₂₀, Aryl oder Heteroaryl ist, wobei vorzugsweise R₉, R₁₁, R₁₃, R₁₅, R₁₇, R₁₉ = H und R₁₀, R₁₂, R₁₄, R₁₆, R₁₈, R₂₀ = Alkyl mit C₁-C₂₀, Cycloalkyl mit C₃-C₂₀, Alkenyl mit C₂-C₂₀, Alkinyl mit C₂-C₂₀, Aryl, Heteroaryl,
- NR²¹R²¹ oder -OR²¹ ist, oder
- bei den Strukturelementen c und/oder d C durch Si ersetzt sein kann, oder
- wahlweise a oder b oder e oder f NR²² ist, mit R²² = Alkyl mit C₁-C₂₀, Cycloalkyl mit C₃-C₂₀, Alkenyl mit C₂-C₂₀, Alkinyl mit C₂-C₂₀, Aryl, Heteroaryl, oder
- wahlweise a und f oder b und e NR sind, mit R²² = Alkyl mit C₁-C₂₀, Cycloalkyl mit C₃-C₂₀, Alkenyl mit C₂-C₂₀, Alkinyl mit C₂-C₂₀, Aryl, Heteroaryl,
- wobei die Bindungen a-c, b-d, c-e und d-f, aber nicht gleichzeitig a-c und c-e und nicht gleichzeitig b-d und d-f, ungesättigt sein können,
- wobei die Bindungen a-c, b-d, c-e und d-f Teil eines gesättigten oder ungesättigten Ringsystems sein können, welches auch die Heteroelemente O, S, Se, N, P, Se, Ge, Sn enthalten kann, oder
- die Bindungen a-c, b-d, c-e und d-f Teil eines aromatischen oder kondensierten aromatischen Ringsystems sind, welches auch die Heteroelemente O, S, Si, N enthalten kann,
- wobei das Atom E ein Hauptgruppenelement ist, bevorzugt ausgewählt aus der Gruppe C, N, P, As, Sb,
- wobei das Strukturelement a-e-b wahlweise Bestandteil eines gesättigten oder ungesättigten Ringsystems ist, welches auch die Heteroelemente O, S, Se, N, P, Si, Ge, Sn enthalten kann, oder
- das Strukturelement a-E-b wahlweise Bestandteil eines aromatischen Ringsystems ist, welches auch die Heteroelemente O, S, Se, N enthalten kann.

Besonders bevorzugt ist, daß der Dotand die folgende Struktur **Ia** aufweist:

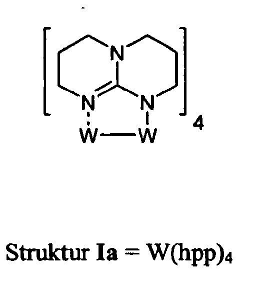

Bevorzugt ist alternativ auch, daß der Dotand eine organische Verbindung mit ausreichend hohem Oxidationspotential (HOMO) ist.

Bevorzugt ist alternativ auch, daß der Dotand ein Alkali- und/oder Erdalkalimetall ist, vorzugsweise Cäsium.

Auch wird vorgeschlagen, daß das erfindungsgemäße organische Halbleitermaterial ein Energieniveau für das unterste unbesetzte Molekülorbital (LUMO) aufweist, welches sich um 0 bis 0,5V gegenüber dem Ionisationspotential (HOMO) des Dotanden, bevorzugt um 0 bis 0,3V, besonders bevorzugt um 0 bis 0,15V, unterscheidet.

Eine Ausführungsform zeichnet sich dadurch aus, daß das Matrixmaterial ein LUMO-Energieniveau aufweist, welches tiefer liegt als das HOMO des Dotanden. "Tiefer" bedeutet in diesem Fall, dass das LUMO-Energieniveau einen kleineren Zahlenwert aufweist als das HOMO-Energieniveau. Da beide Größen negativ vom Vakuumniveau aus angegeben werden, bedeutet dies, dass der absolute Betrag des HOMO-Energiewertes kleiner ist als der absolute Betrag des LUMO-Energiewertes.

Bevorzugt ist auch, daß die Konzentration des Dotanden 0,5 bis 25 Masseprozent, bevorzugt 1 bis 15 Masseprozent, besonders bevorzugt 2,5 bis 5 Masseprozent beträgt.

Erfindungsgemäß sind auch eine organische Leuchtdiode, welche ein erfindungsgemäßes Halbleitermaterial umfaßt, sowie 5,6-Dihydrophenanthroline der Struktur 7.

Überraschenderweise wurde festgestellt, daß asymmetrisch substituierte Phenanthroline als Matrixmaterialien eingesetzt werden können, die mit Metallkomplexdotanden dotiert werden können, insbesondere mit denen der Struktur I. Durch Einsatz solcher dotierten Schichten erhöht sich die Leistungseffizienz einer erfindungsgemäßen OLED.

Die erfindungsgemäß für das Halbleitermaterial eingesetzten Matrixmaterialien zeigen ferner eine gegenüber dem Stand der Technik verbesserte thermische Stabilität, die insbesondere auf erhöhte Glasübergangstemperaturen und erhöhte Sublimations- und Zersetzungstemperaturen zurückzuführen ist.

Bevorzugt ist, dass die Glasübergangstemperatur Tg des Matrixmaterials höher ist als diejenige von 4,7-Diphenyl-1,10-phenanthrolin (Tg = 63°C).

Bevorzugt weist das Matrixmaterial eine Verdampfungstemperatur von mindestens 200°C auf.

Bevorzugt weist das Matrixmaterial eine Zersetzungstemperatur von mindestens 300°C auf.

Asymmetrische Phenanthroline, wie sie oben definiert werden, sind aus der Literatur bisher nicht bekannt.

2,4-Disubstituierte Hydrochinolinone der Struktur **4** lassen sich in einer Dreistufensynthese, ausgehend von 2,4,6-Triarylpyryliumsalzen **1** und Cyclohexandion, über die Stufe der 8-Oxo-1-benzopyryliumsalze **3** gewinnen (s. Schema 1). 8-Oxo-1-benzopyryliumperchlorat wurde bereits von Zimmermann et al. beschrieben (T. Zimmermann, G.W. Fischer, B. Olk, M. Findeisen: J. Prakt. Chem. 331, 306-318 [1989]). Da die Verwendung von Perchloraten in größerem Maßstab aus Sicherheitsgründen problematisch ist, wurde folgende Synthese über das Tetrafluoroborat **3** neu entwickelt.

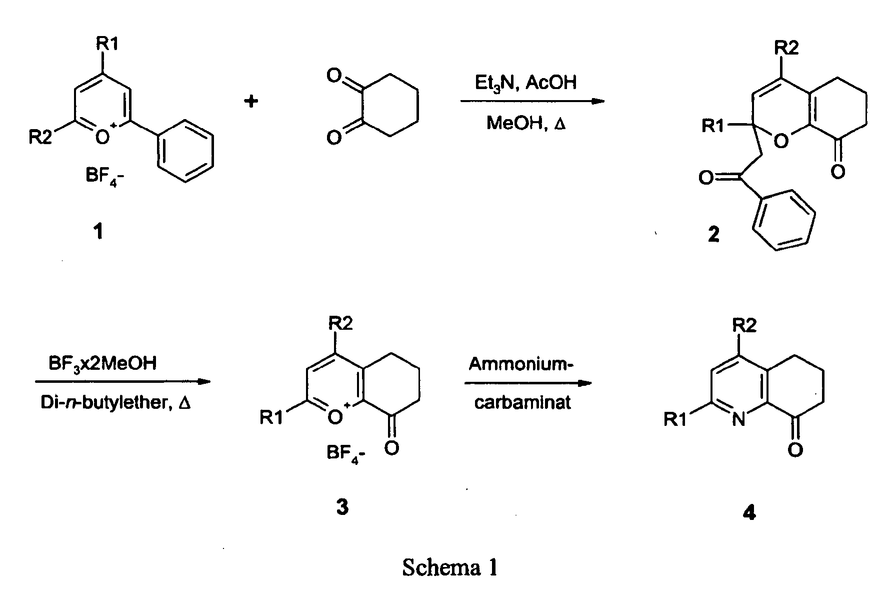

2,4-disubstituierte Hydrochinolinone der Struktur **4** haben sich dabei als besonders wertvolle Synthesebausteine für den Aufbau von N-Heterozyklen erwiesen. Es ist bekannt, dass die Kondensation von Chinolinonen und Aldehyden zur Bildung von substituierten Methylenderivaten herangezogen werden kann. Die Anwendung dieser Reaktion auf die 2,4-disubstituierten Hydrochinolinone nach Schema 2 führt zu substituierten Methylenhydrochinolinonen **5**.

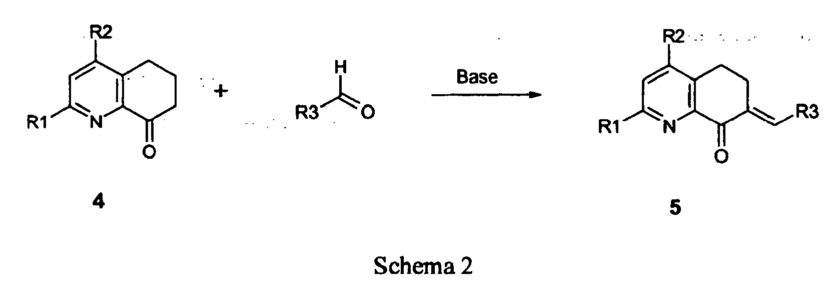

Es hat sich gezeigt, dass diese Substanzklasse die Schlüsselverbindung zur Darstellung der asymmetrisch substituierten Phenanthroline **8** darstellt, welche Hauptgegenstand der vorliegenden Erfindung sind.

Es wurde überraschend gefunden, dass die Umsetzung der genannten substituierten Methylenhydrochinolinone **5** mit verschiedenen Enaminen unter basischen Bedingungen zur Bildung der Zwischenverbindungen **6** führt, welche anschließend durch Oxidation in neuartige 5,6-Dihydrophenanthroline **7** nach Schema 3 übergeführt werden können.

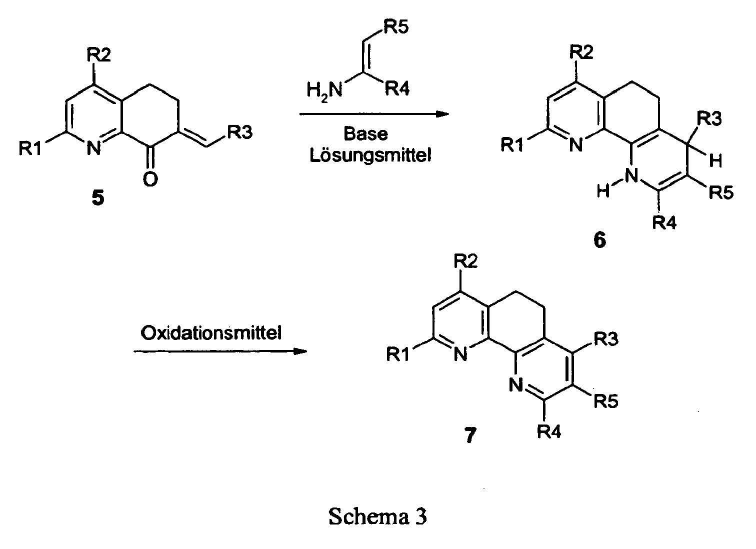

Die anschließende Aromatisierung zu den asymmetrisch substituierten Phenanthrolinen **8** kann bspw. durch Pd-katalysierte Dehydrierung der Dihydroverbindungen **7** nach Schema **4** erfolgen.

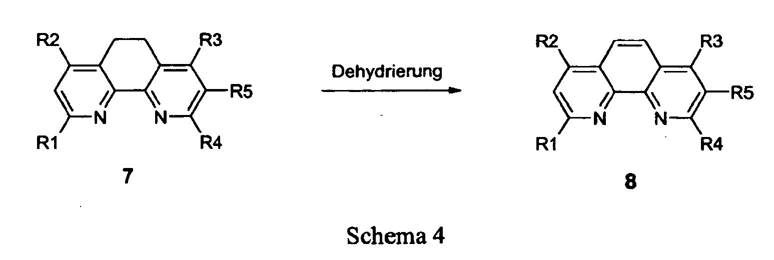

Weitere Merkmale und Vorteile der Erfindung ergeben sich aus der nachfolgenden detaillierte Beschreibung bevorzugter Ausführungsbeispiele.

### Beispiel 1

Es wird die vollstänge Synthese eines Derivates **8a** der Substanzklasse der 2,4,7,8,9-pentasubstituierten asymmetrischen Phenanthroline **8** im Folgenden beschrieben.

### Stufe 1: Synthese von 5,6-Dihydro-2-phenacyl-2,4-diphenyl-2H,7H-1-benzopyran-8-on 2a

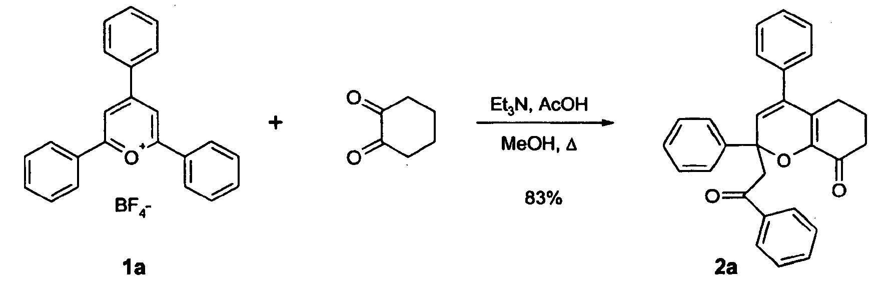

**Lit.:** T. Zimmermann, G.W. Fischer, B. Olk, M. Findeisen: J. prakt. Chem. 331, 306-318 [1989]
Eine Suspension von 13,51g 2,4,6-Triphenylpyryliumtetrafluoroborat (34,1mmol) und 4,78g Cyclohexan-1,2-dion (42,6mmol) wird in Methanol zum Sieden erhitzt, dann wird eine Lösung von 4,56g Essigsäure (75,9mmol) und 7,68g Triethylamin (75,9 mmol) in wenig Methanol hinzugetropft. Nach 7 h Erhitzen unter Rückfluss lässt man auf Raumtemperatur abkühlen. Der hellgelbe Niederschlag wird abgesaugt, portionsweise mit 100 ml eisgekühltem Methanol gewaschen und bis zur Gewichtskonstanz getrocknet. Man erhält 11,37 g eines hellgelben Pulvers (79,3%) mit einem Schmelzpunkt von 178 - 179°C (Lit.: 182 - 184°C).

### Stufe 2: Synthese von 5,6,7,8-Tetrahydro-8-oxo-2,4-diphenyl-1-benzopyryliumtetrafluoroborat 3a

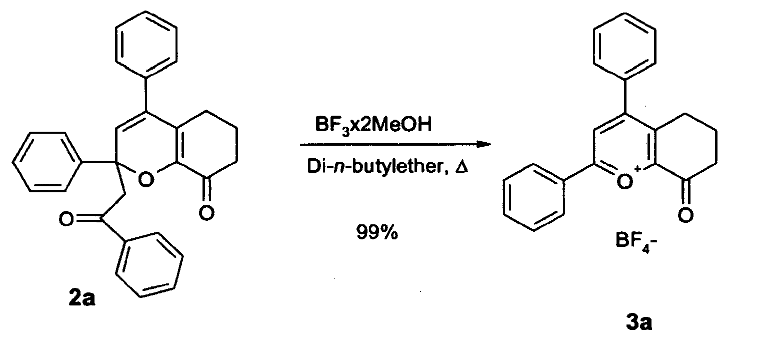

Die Suspension von 32,7g 5,6-Dihydro-2-phenacyl-2,4-diphenyl-*2H,7H*-1-benzopyran-8-on (77,8mmol) in 500ml Di-*n*-butylether wird mit 25ml BF₃-Dimethanol-Komplex (50% BF₃) versetzt, wobei sich die Suspension orange färbt. Die Suspension wird 2 h bei Raumtemperatur nachgerührt, anschließend zum Sieden erhitzt und 2 h unter Rückfluss gekocht. Nach Abkühlen auf Raumtemperatur wird der Niederschlag über eine Nutsche mit Papierfilter abgesaugt, mit Diethylether gewaschen. Nach Trocknen im Hochvakuum erhält man 29,9g (99%) terracottafarbenes Pulver mit einem Schmelzpunkt von 213-214°C (Zersetzung). IR: Die CO-Valenz-Schwingungsbande erscheint bei 1718 cm⁻¹.

### Stufe 3: Synthese von 6,7-Dihydro-2,4-diphenylchinolin-8(5H)-on 4a

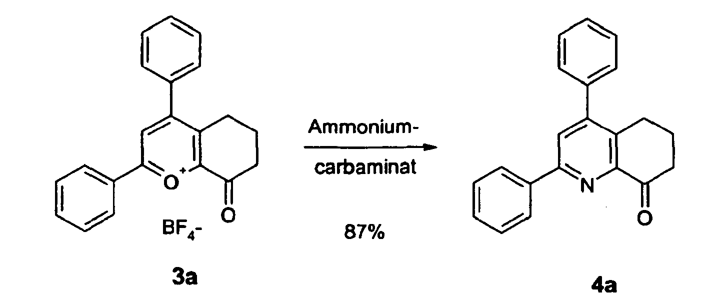

Es werden 12,44g 5,6,7,8-Tetrahydro-8-oxo-2,4-diphenyl-1-benzopyrylium-tetrafluoroborat (32mmol) in 80ml destilliertem Wasser suspendiert und unter Rühren mit einer Lösung von 25g Ammoniumcarbaminat (320mmol) in 200 ml destilliertem Wasser versetzt. Nach 24h Rühren bei Raumtemperatur wird über eine Nutsche abgesaugt, mit destilliertem Wasser gewaschen und trocken gesaugt. Das cremefarbene Pulver wird portionsweise mit Diethylether gewaschen und bei 50°C im Vakuumtrockenschrank bis zur Gewichtskonstanz getrocknet: 8,39 g elfenbeinfarbenes Produkt (87,5%) mit einem Schmelzpunkt von 177-178°C (Zersetzung). IR (ATR): Die CO-Valenz-Schwingungsbande ist bei 1700 cm⁻¹ zu sehen. ¹H-NMR (500 MHz, CDCl₃): δ = 8,09 (d, 2H, o-H von 2-Phenyl), 7,79 (s, 1H, H₃), 7,56 - 7,33 (m, 8H, arom. H), 2,92 (t, 2H, *J* = 5,75 Hz), 2,83 (t, 2H, *J* = 6,56 Hz), 2,10 (p, 2H, *J* = 5,75 Hz, *J* = 6,56 Hz).

### Stufe 4: Synthese von 7-Benzyliden-6,7-dihydro-2,4-diphenyl-chinolin-8(5H)-on 5a

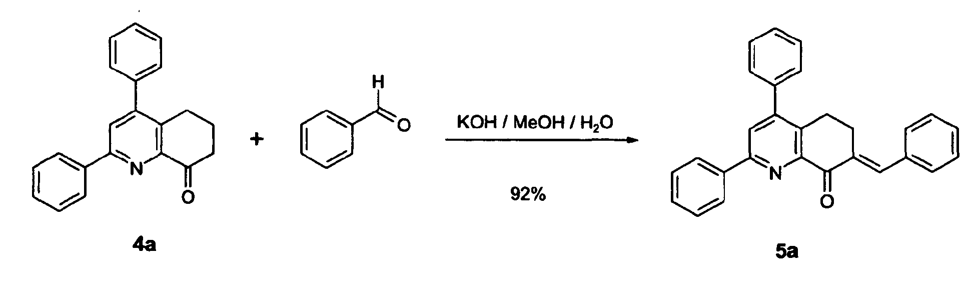

Zur Suspension von 13,8 g 6,7-Dihydro-2,4-diphenyl-chinolin-8(*5H*)-on (46 mmol) und 5,9 g Benzaldehyd (55mmol) in 200 ml Methanol wird unter gutem Rühren die Lösung von 5,6 g Kaliumhydroxid (100mmol) in 40ml destilliertem Wasser hinzugefügt. Die cremefarbene Suspension wird nach 1d Rühren bei Raumtemperatur mit 8ml Eisessig neutralisiert und 30min bei Raumtemperatur nachgerührt. Das elfenbeinfarbene Produkt wird abgesaugt, mit 100 ml destilliertem Wasser gewaschen, scharf trocken gesaugt und luftgetrocknet. Das elfenbeinfarbene Pulver wird in 80 ml Methanol suspendiert und 30min bei Raumtemperatur nachgerührt. Der Niederschlag wird abgesaugt, mit wenig Methanol sowie Diethylether gewaschen, trocken gesaugt und bei 60°C im Vakuumtrockenschrank bis zur Gewichtskonstanz getrocknet. Man erhält 16,38g eines elfenbeinfarbenen Pulvers (91,9%) mit einem Schmelzpunkt von 185 - 187°C (Zersetzung). Das Produkt zeigt im IR Spektrum die für Chalkone charakteristische CO-Valenzschwingungsbande bei 1675 cm⁻¹. ¹H-NMR (500 MHz; CD₂Cl₂): δ = 8,13 (d, 2H, o-H von 2-Phenyl), 7,93 (s, 1H, H₉), 7,87 (s, 1H, H₃), 7,56-7,32 (m, 13 arom. H), 3,09 (dt, 2H), 2,95 (t, 2H).

### Stufe 5: Synthese von 1,4,5,6-Tetrahydro-2-methyl-4,7,9-triphenyl-1,10-phenanthrolin 6a

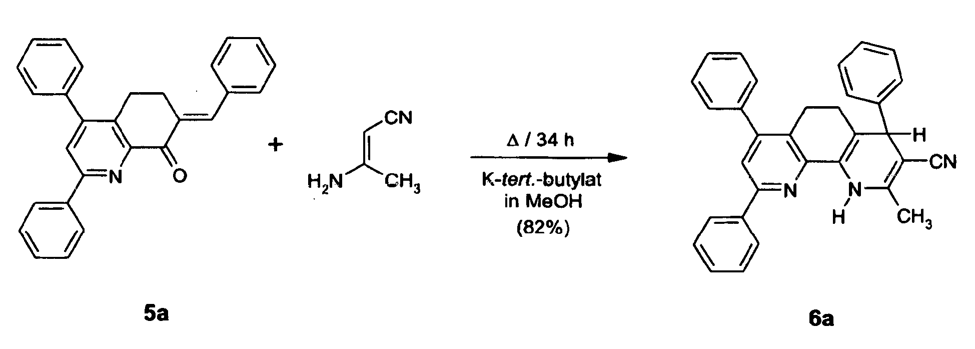

Es werden 1,55 g 7-Benzyliden-6,7-dihydro-2,4-diphenyl-chinolin-8(5H)-on **5a** (4 mmol) und 0,41 g 3-Amino-crotonsäurenitril (5 mmol) in 50 ml Acetonitril suspendiert und durch leichtes Erwärmen gelöst. Zu der resultierenden bräunlich-gelben Lösung wird in kleinen Portionen ohne weitere Wärmezufuhr die Lösung von 1,12 g Kalium-tert.-butylat (10 mmol) in 10 ml Methanol hinzugefügt, wobei eine intensiv gelbe Lösung entsteht, aus der sich allmählich ein hellgelber, feinkristalliner Niederschlag abscheidet. Die Suspension wird nochmals zum Sieden erhitzt und 34 h (DC Kontrolle) unter Rückfluss gekocht. Nach Abkühlen auf Raumtemperatur zeigt das DC (Aluminiumoxid; Laufmittel: Methylenchlorid) der überstehenden braungelben Reaktionslösung, dass nur noch Spuren des Edukts enthalten sind. Die Suspension wird unter Eiswasserkühlung tropfenweise mit 2 ml Eisessig neutralisiert und 30 min im Eiswasserbad nachgerührt. Der Niederschlag wird abgesaugt, mit 100 ml destilliertem Wasser sowie mit 20 ml Methanol gewaschen und scharf trocken gesaugt. Man erhält 1,47 g (81%) hellgelbes, mikrokristalline Substanz mit einem Schmelzpunkt von 245-249°C.
¹H-NMR (500 MHz; CD₂Cl₂): δ = 8,05 (d, 2H, o-H von 9-Phenyl), 7,71 (s, NH, breit), 7,59 (s, 1H, H₈) 7,55 - 7,24 (m, 13 arom. H), 4,33 (s, 1H, H₄), 2,89 - 2,72 (m, 2H), 2,27 (s, 3H, CH₃), 2,18 - 2,09 (m, 1H), 2,07 - 1,96 (m, 1H).

### Stufe 6: Synthese von 5,6-Dihydro-2-methyl-4,7,9-triphenyl-1,10-phenanthrolin-3-carbonitril 7a

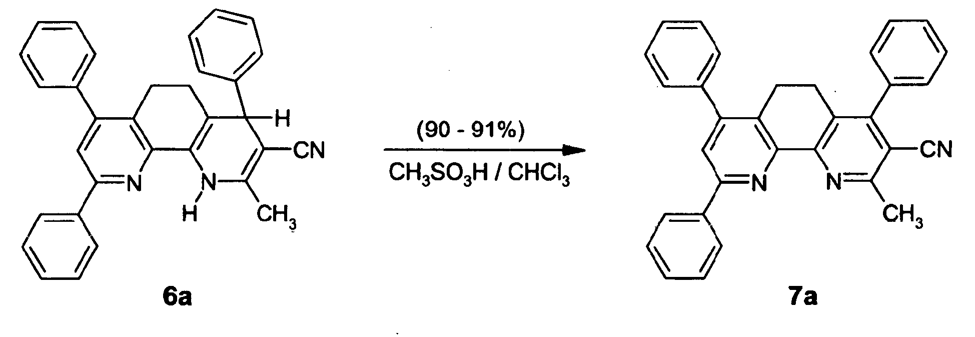

Zu einer Lösung von 1,5 g 1,4,5,6-Tetrahydro-2-methyl-4,7,9-triphenyl-1,10-phenanthrolin-3-carbonitril (3,21 mmol) in 20 ml Chloroform werden bei Raumtemperatur 1,5 ml Methansulfonsäure tropfenweise hinzufügt. Die orangegelbe Lösung wird 30 min unter Rückfluss erhitzt und nach Abkühlen zweimal mit jeweils 40 ml destilliertem Wasser ausgeschüttelt, wobei sich die Chloroformphase hellgelb färbt. Die Chloroformphase wird über wasserfreiem Kaliumcarbonat getrocknet und am Rotavapor unter vermindertem Druck zur Trockene eingeengt. Der grüngelbe, ölige Rückstand wird in 10 ml Dichloromethan aufgenommen und mit 50 ml Cyclohexan versetzt. Nach Abzug des Dichlormethans beginnt sich nach kurzer Zeit ein hellgelbes, mikrokristallines Produkt abzuscheiden. Der hellgelbe Niederschlag wird abgesaugt, mit 20 ml Hexan gewaschen und im Vakuumtrockenschrank bei 60 °C bis zur Gewichtskonstanz getrocknet. Man erhält 1,31 g (90,8%) eines hellgelben Pulvers mit einem Schmelzpunkt von 244 - 246°C.
**¹H-NMR** (500 MHz, CD₂Cl₂): δ = 8,15 (d, 2H, o-H von 2-Phenyl), 7,78 (s, H₃), 7,60 - 7,31 (m, 13H, arom. H), 2,97 (s, 3H, CH₃), 2,87 (td, 2H), 2.70 (td, 2H)
Laut TGA ist die Verbindung bis 340°C thermostabil (5%-Wert). Die Glasübergangstemperatur beträgt laut DSC 97 °C. Das UV/VIS-Spektrum in CH₂Cl₂ zeigt eine nach längerer Wellenlänge hin leicht strukturierte Bande bei 251 nm sowie eine Bande mit geringerer Intensität bei 342 nm. Das Fluoreszenzspektrum zeigt bei beiden Anregungswellenlängen eine Emissionsbande bei 391 nm. Aufgrund der Röntgenstrukturanalyse wird bestätigt, dass 5,6-Dihydro-2-methyl-4,7,9-triphenyl-1,10-phenanthrolin-3-carbonitril vorliegt und nicht das strukturisomere 5,6-Dihydro-10-methyl-2,4,7-triphenyl-1,9-phenanthrolin-8-carbonitril.
5,6-Dihydro-2-methyl-4,7,9-triphenyl-1,10-phenanthrolin-3-carbonitril **7a** besitzt in Acetonitril vs. Fc/Fc+ für die Reduktion ein 1. Halbstufenpotential von E_{1/2I,red} = -2,05 [V] (reversibel) sowie ein 2. Halbstufenpotential von E_{1/2II,red} = -2,51 [V] (irreversibel). Das Oxydationspotential vs. Fc/Fc+ liegt bei Eox = 1,52 [V] (irreversibel).

### Stufe 7: Synthese von 2-Methyl-4,7,9-triphenyl-1,10-phenanthrolin-3-carbonitril 8a

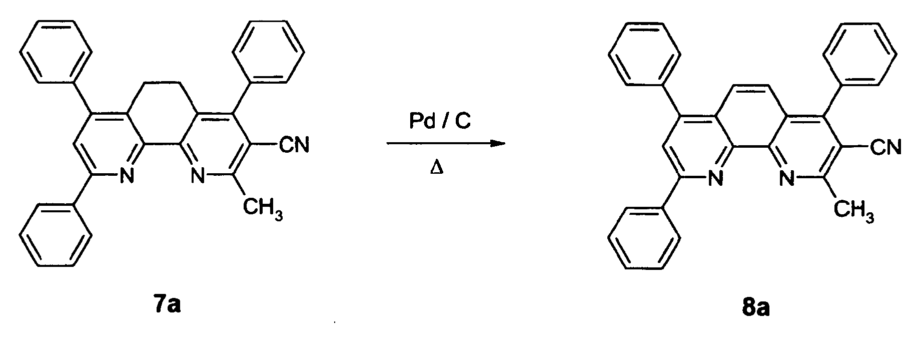

Es werden 1,5 g 5,6-Dihydro-2-methyl-4,7,9-triphenyl-1,10-phenanthrolin-3-carbonitril (3,34 mmol) **7a** in 75 ml Diethylenglykol unter Erwärmen gelöst, portionsweise mit 270 mg Pd / C (5%) versetzt, zum Sieden erhitzt und 53 h Kochen unter Rückfluss gekocht. Man lässt die schwarze Suspension an der Luft unter gutem Rühren auf Raumtemperatur abkühlen. Nach Zufügen von 100 ml destilliertem Wasser wird die Suspension 30 min bei Raumtemperatur nachgerührt und über eine D3-Glasfritte filtriert. Der Rückstand wird portionsweise mit 200 ml destilliertem Wasser gewaschen und scharf trocken gesaugt. Das milchig trübe Filtrat wird mit Kochsalz gesättigt, zweimal mit jeweils 25 ml Chloroform ausgeschüttelt und verworfen. Der auf der Glasfritte verbliebene schwarze Rückstand wird auf der Fritte portionsweise mit 400 ml Chloroform gewaschen. Die vereinigten grünlich-gelben Chloroformlösungen werden über wasserfreiem Natriumsulfat getrocknet, filtriert und am Rotavapor zur Trockene eingedampft. Der braungelbe, ölige Rückstand wird in 50 ml Diethylether aufgenommen, wobei sich ein grüngelber, mikrokristalliner Niederschlag abscheidet, der über eine Nutsche mit Papierfilter abgesaugt und mit 20 ml Diethylether gewaschen wird. Man erhält 1,04 g (70%) eines gelbgrünen, mikrokristallinen Produkts (R_{f} = 0,5; Aluminiumoxid; Laufmittel Dichloromethan). Nach Umkristallisieren aus EtOH / CHCl₃ erhält man ein nadelförmig kristallisierendes Produkt, welches laut ¹H-NMR (CD₂Cl₂₎ das gewünschte 2-Methyl-4,7,9-triphenyl-1,10-phenanthrolin-3-carbonitril ist: δ = 8,38 (d, 2 H, *o*-H von 9-Phenyl), 8,14 (s, 1 H, H₈), 7,85 (d, 1 H, *J* = 9,3 Hz) und 3,20 (s, 3 H, CH₃). Das IR-Spektrum (ATR) zeigt die CN-Bande bei 2277 cm⁻¹. Die in geringer Intensität zu beobachtende Bande bei 1617 cm⁻¹ ist auf das Phenanthrolinsystem zurückzuführen.
2-Methyl-4,7,9-triphenyl-1,10-phenanthrolin-3-carbonitril **8a** besitzt in MeCN das Reduktionshalbstufenpotential E_{1/2}^{I,red} = -2,1 V vs. Fc/Fc⁺ (reversibel). Ein Oxidationspotential ist innerhalb des in MeCN zur Verfügung stehenden Fensters nicht messbar.

Eine Zersetzungstemperatur von **8a** (TGA) unter Stickstoff konnte bis 400°C nicht festgestellt werden. Somit ist die Verbindungsklasse thermisch stark belastbar. Die Sublimationstemperatur von **8a** beträgt 238°C und befindet sich damit deutlich über der 200°C Marke. Die Glasübergangstemperatur von **8a** beträgt 93°C.

Aus einem Vergleich der Glasübergangstemperaturen von **7a** und **8a** mit der von **BPhen** ergibt sich, dass die morphologische Stabilität von BPhen sehr gering, hingegen die von **7a** und **8a** deutlich erhöht ist.

### Beispiel 2

Es wird die partielle Synthese eines Derivates **8b** der neuen Substanzklasse der 2,4,7,8,9-pentasubstituierten Phenanthroline **8** im Folgenden beschrieben. Die Synthese der Stufen 1-4 erfolgte analog zum Beispiel 1.

### Stufe 5: Synthese von 1,4,5,6-Tetrahydro-2,4,7,9-tetraphenyl-1,10-phenanthrolin-3-carbonitril

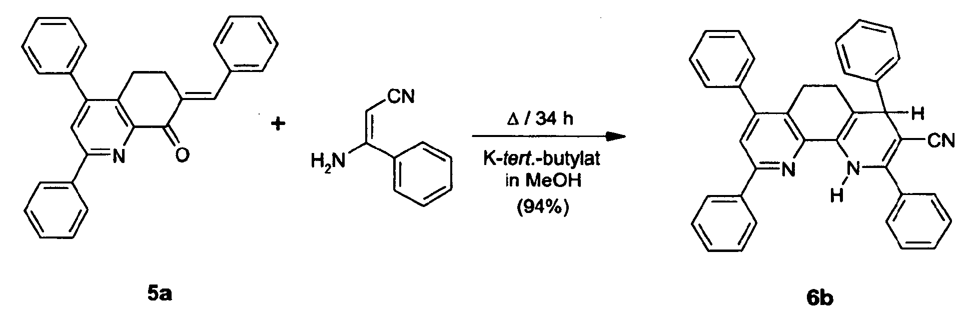

Es werden 4,03 g 7-Benzyliden-6,7-dihydro-2,4-diphenyl-chinolin-8(5*H*)-on (10,4 mmol) und 1,73 g 3-Amino-3-phenyl-acrylnitril (12,0 mmol) in 40 ml Ethanol suspendiert und zum Sieden erhitzt. Unter gutem Rühren wird in der Siedehitze inert über 8 min eine Lösung von 2,24 g Kalium-*tert*.-butylat (20 mmol) in 20 ml Ethanol hinzugetropft. Die entstehende Suspension wird 21 h am Rückfluss erhitzt und anschließend unter kräftigem Rühren an der Luft auf Raumtemperatur abgekühlt.
Die Suspension wird unter Eiswasserkühlung tropfenweise mit 4 ml Eisessig neutralisiert und 30 min im Eiswasserbad nachgerührt. Der Niederschlag wird abgesaugt, mit 100 ml destilliertem Wasser sowie mit 20 ml Ethanol gewaschen und bei 80 °C im Vakuumtrockenschrank bis zur Gewichtskonstanz getrocknet. Man erhält 5,01 g (94 %) hellgelbes, mikrokristallines Pulver mit einem Schmelzpunkt von 233 - 236 °C. **¹H-NMM** (500 MHz; CD₂Cl₂): δ = 8,05 - 7,97 (m, 2H, *o*-H von 9-Phenyl + NH), 7,76 - 7,70 (m, 2H, *o*-H von 2-Phenyl), 7.61 (s, 1H, H₈) 7,57 - 7,51 (m, 3 arom. H), 7,51 - 7,34 (m, 12 arom. H), 7,34 - 7,28 (m, 1 arom. H), 4,48 (s, 1H, H₄), 2,96 - 2,78 (m, 2H), 2,27 - 2,17 (m, 1H), 2,17 - 2,07 (m, 1H); IR (ATR): NH-Valenzschwingungsbande bei 3401 cm⁻¹, Cyanobande bei 2190 cm⁻¹.

### Stufe 6: Synthese von 2,4,7,9-Tetraphenyl-1,10-phenanthrolin-3-carbonitril 8b

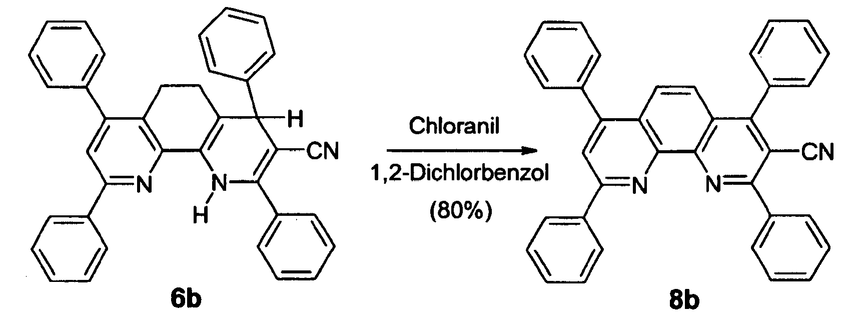

Es werden 5,34 g 1,4,5,6-Tetrahydro-2,4,7,9-tetraphenyl-1,10-phenanthrolin-3-carbonitril (10 mmol) und 5,16 g Chloranil (21 mmol) in 25 ml 1,2-Dichlorobenzol suspendiert und unter gutem Rühren zum Sieden erhitzt. Die resultierende gelbbraune Lösung wird 24 h am Rückfluss erhitzt, dann lässt man unter gutem Rühren an der Luft auf Raumtemperatur abkühlen, wobei ein Kristallbrei entsteht. Dieser wird in 50 ml Ethanol aufgenommen, zum Sieden erhitzt und 30 min am Rückfluss erhitzt. Nach Abkühlen auf Raumtemperatur wird der bräunliche Niederschlag über eine Nutsche mit Papierfilter abgesaugt, mit 50 ml Ethanol sowie mit 50 ml Diethylether gewaschen und scharf getrocknet. Das Rohprodukt wird in 300 ml siedendem Chloroform gelöst, mit 5 g wasserfreiem Kaliumcarbonat versetzt, 30 min am Rückfluss erhitzt und heiß über ein Faltenfilter in 300 ml Ethanol filtriert, wobei sich ein weißer, voluminöser, mikrokristalliner Niederschlag abscheidet, der über eine Nutsche mit Papierfilter abgesaugt und mit 100 ml Ethanol gewaschen wird. Nach Trocknen im Vakuumtrockenschrank bei 80 °C bis zur Gewichtskonstanz erhält man 4,08 g elfenbeinfarbenes Pulver mit einem Schmelzpunkt von 343 - 344 °C.
**¹H-NMR** (500 MHz; CDCl₃): δ = 8,43 (d, 2H, o-H von 2-Phenyl), 8,30 (d, 2H, o-H von 9-Phenyl), 8,15 (s, 1H, H₈) 7,89 (d, 1H, *J* = 9,28 Hz, H₅), 7,67 - 7,47 (m, 17H, 16 arom. H + H₆).
2,4,7,9-Tetraphenyl-1,10-phenanthrolin-3-carbonitril 8b besitzt in MeCN ein Reduktionshalbstufenpotential E_{1/2}^{I,red} = -2,04V vs. Fc/Fc⁺ (reversibel). Ein Oxidationspotential ist innerhalb des in MeCN zur Verfügung stehenden Fensters nicht messbar.

Eine Zersetzungstemperatur von **8b** (TGA) unter Stickstoff konnte bis 400°C nicht festgestellt werden. Somit ist die Verbindungsklasse thermisch stark belastbar. Die Sublimationstemperatur von **8b** beträgt 282°C und befindet sich damit deutlich über der 200°C Marke. Die Glasübergangstemperatur von **8b** beträgt 118°C.
Aus einem Vergleich der Glasübergangstemperaturen von **7b** und **8b** mit der von **BPhen** ergibt sich, dass die morphologische Stabilität von BPhen sehr gering, hingegen die von **7b** und **8b** deutlich erhöht ist.

### Beispiel 3

Ein Glassubstrat wird mit Kontakten aus Indium-Zinn-Oxid versehen. Auf das Substrat wird danach eine Schicht aus **8a** dotiert mit einem Dotanden der Struktur **Ia** hergestellt. Die Dotierkonzentration des Dotanden **Ia** beträgt 10mol%. Die Leitfähigkeit der Schicht beträgt 2,99E-5 S/cm bei Raumtemperatur.

### Beispiel 4

Ein Glassubstrat wird mit Kontakten aus Indium-Zinn-Oxid versehen. Auf das Substrat wird danach eine Schicht aus **8b** dotiert mit einem Dotanden der Struktur **Ia** hergestellt. Die Dotierkonzentration des Dotanden **Ia** beträgt 10mol%. Die Leitfähigkeit der Schicht beträgt 1,38E-5 S/cm bei Raumtemperatur.

### Vergleichsbeispiel 5

Ein Vergleichsbeispiel 5 wurde analog zur Vorgehensweise nach Beispielen 3 und 4 durchgeführt, jedoch wurde anstelle des Matrixmaterials **8a** das aus der Literatur bekannte **BPhen** verwendet. Die erhaltene Leitfähigkeit der Schicht beträgt 4,12E-9 S/cm.

Ein Vergleich der Leitfähigkeiten aus Beispielen 3 und 4 mit den Leitfähigkeitswerten für die aus dem Stand der Technik bekannten Matrixmaterialien Alq₃, BPhen oder BAlq₃ (Tabelle 1 und Vergleichsbeispiel 5) zeigt somit für die erfindungsgemäße Substanzklasse **8** eine deutliche, um mindestens 3 Größenordnungen verbesserte Leitfähigkeit.

### Beispiel 6

Ein Substrat aus Glas wird mit Indium-Zinn-Oxid-Kontakten versehen. Darauf werden dann nacheinander die Schichten: Spiro-TTB dotiert mit p-Dotand 2-(6-Dicyanomethylene-1,3,4,5,7,8-hexafluoro-6H-naphthalen-2-yliden)-malononitril (50nm, 1,5 w%) / N,N'-Di(naphthalen-2-yl)-N,N'-diphenylbenzidin (10nm) / N,N'-Di(naphthalen-2-yl)-N,N'-diphenylbenzidin dotiert mit Emitter Iridium(III)bis(2-methyldibenzo-[f,h]quinoxalin)(acetylacetonat) (20nm, 10wt%) / 2,4,7,9-Tetraphenylphenanthrolin (10nm) / **8a** dotiert mit **Ia** (60nm, 8 w%) / A1 (100nm) abgeschieden. Die so hergestellte organische Leuchtdiode emittiert orangerotes Licht und weist bei 1000 cd/m² folgende Betriebsparameter auf: 3,0 V und 28,2 cd/A.

### Beispiel 7

Ein Substrat aus Glas wird mit Indium-Zinn-Oxid-Kontakten versehen. Darauf werden dann nacheinander die Schichten: Spiro-TTB dotiert mit p-Dotand 2-(6-Dicyanomethylene-1,3,4,5,7,8-hexafluoro-6H-naphthalen-2-yliden)-malononitril (50nm, 1,5 w%) / N,N'-Di(naphthalen-2-yl)-N,N'-diphenylbenzidin (10nm), N,N'-Di(naphthalen-2-yl)-N,N'-diphenylbenzidin dotiert mit Emitter Iridium(III)bis(2-methyldibenzo-[f,h]quinoxalin)(acetylacetonat) (20nm, 10wt%) / 2,4,7,9-Tetraphenylphenanthrolin (10nm) / **8b** dotiert mit **Ia** (60nm, 8 w%) / A1 (100nm) abgeschieden. Die so hergestellte organische Leuchtdiode emittiert orangerotes Licht und weist bei 1000 cd/m² folgende Betriebsparameter auf: 3,1 V und 26,8 cd/A.

### Vergleichsbeispiel 8

### (Stand der Technik)

Eine OLED Struktur wie in den Beispielen 5 und 6 wurde hergestellt. Dabei wurde statt der mit **Ia** n-dotierten Elektronentransportschicht **8a** die undotierte Elektronentransportschicht **8a** verwendet. Diese OLED weist bei 1000 cd/m² folgende Betriebsparameter auf: 6,8V und 17,2 cd/A.

Die Verwendung von mit **Ia** dotiertem **8a** als Elektronentransportschicht führt also dazu, dass eine OLED Struktur eine geringere Betriebsspannung und/ oder eine höhere Stromeffizienz aufweist. Mithin ist auch die Leistungseffizienz des Bauelementes erhöht.

Die in der Beschreibung und in den Ansprüchen offenbarten Merkmale können sowohl einzeln als auch in einer beliebigen Kombination zur Verwirklichung der Erfindung in ihren unterschiedlichen Ausführungsformen wesentlich sein.

## Patentansprüche

1. Asymmetrisch substituierte Phenanthroline der folgenden Struktur **8**: 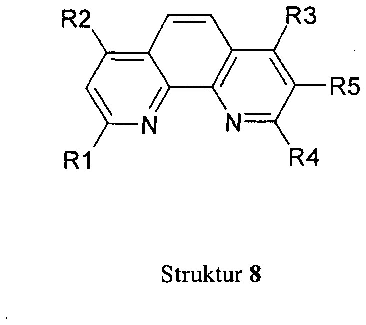 wobei:
R¹ und R² unabhängig ausgewählt sind aus Aryl und Heteroaryl;
R³ Aryl oder Heteroaryl ist;
R⁴ Aryl, Heteroaryl C₁ - C₂₀ Alkyl oder C₃ - C₂₀ Cycloalkyl ist;
R⁵ CN, COOAlkyl, COAryl oder COAlkyl ist.

2. Verfahren zur Herstellung von asymmetrisch substituierten Phenanthrolinen, welches die folgenden Schritte umfaßt:
(ia) Umsetzung eines substituierten Methylenhydrochinolinons **5** mit Enamin
unter basischen Bedingungen zur Darstellung eines 1,4,5,6-Tetrahydrophenanthrolins **6** und anschließende Oxidation zur Darstellung
e
eines 5,6-Dihydrophenanthrolins **7** gemäß dem folgenden Reaktionsschema: 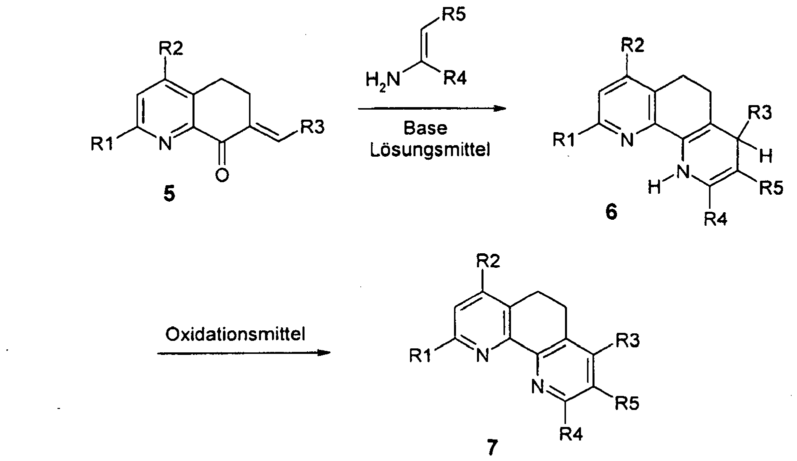
(iia) Aromatisierung des 5,6-Dihydrophenanthrolins **7** zum asymmetrisch substituierten Phenanthrolin **8** gemäß dem folgenden Reaktionsschema: 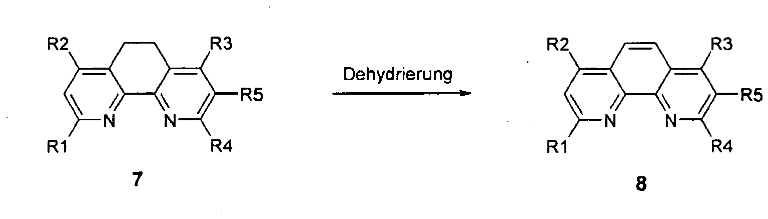 oder
(ib) Umsetzung eines substituierten Methylenhydrochinolinons **5** mit Enamin
unter basischen Bedingungen zur Darstellung eines 1,4,5,6-Tetrahydrophenanthrolins **6** und anschließende direkte Oxidation zum asymmetrisch substituierten Phenanthrolin **8**,
wobei R¹, R², R³, R⁴ und R⁵ wie oben in Anspruch 1 definiert sind.

3. Verfahren nach Anspruch 2, **dadurch gekennzeichnet, daß** zur Oxidation des 1,4,5,6-Tetrahydrophenanthrolins **6** in Schritt (i) Methansulfonsäure oder Chloranil eingesetzt wird.

4. Verfahren nach Anspruch 2 oder 3, **dadurch gekennzeichnet, daß** die Aromatisierung in Schritt (ii) durch Pd-katalysierte Dehydrierung mittels Pd/C erfolgt.

5. Dotiertes organisches Halbleitermaterial umfassend mindestens ein organisches Matrixmaterial, das mit mindestens einem Dotanden dotiert ist, wobei das Matrixmaterial ein asymmetrisch substituiertes Phenanthrolin **8** nach Anspruch 1 ist.

6. Halbleitermaterial nach Anspruch 5, **dadurch gekennzeichnet, daß** das Matrixmaterial reversibel reduzierbar ist.

7. Halbleitermaterial nach Anspruch 5, **dadurch gekennzeichnet, daß** das Matrixmaterial bei einer Reduktion in stabile, redox-reaktive Bestandteile zerfällt.

8. Halbleitermaterial nach einem der Ansprüche 5 bis 7, **dadurch gekennzeichnet, daß** der Dotand ein Metallkomplex ist.

9. Halbleitermaterial nach Anspruch 8, **dadurch gekennzeichnet, daß** der Metallkomplex eine Struktur I aufweist: 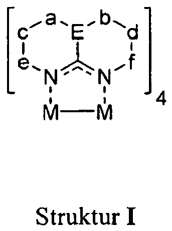 wobei M ein Übergangsmetall ist, vorzugsweise Mo oder W; und wobei
- die Strukturelemente a-f die Bedeutung: a= -CR₉R₁₀-, b = -CR₁₁R₁₂-, c = -CR₁₃R₁₄-, d = R₁₅R₁₆-, e = -CR₁₇R₁₈- und f = -CR₁₉R₂₀- aufweisen können, wobei R₉-R₂₀ unabhängig Wasserstoff, Alkyl mit C₁-C₂₀, Cycloalkyl mit C₃-C₂₀, Alkenyl mit C₁-C₂₀, Alkinyl mit C₂-C₂₀, Aryl, Heteroaryl, -NR²¹R²¹ oder -OR²¹ sind, wobei R²¹ = Alkyl mit C₁-C₂₀, Cycloalkyl mit C₃-C₂₀, Alkenyl mit C₂-C₂₀, Alkinyl mit C₂-C₂₀, Aryl oder Heteroaryl ist, wobei vorzugsweise R₉, R₁₁, R₁₃, R₁₅, R₁₇, R₁₉ = H und R₁₀, R₁₂, R₁₄, R₁₆, R₁₈, R₂₀ = Alkyl mit C₁-C₂₀, Cycloalkyl mit C₃-C₂₀, Alkenyl mit C₂-C₂₀, Alkinyl mit C₂-C₂₀, Aryl, Heteroaryl, -NR¹¹R²¹ oder -OR²¹ ist, oder
- bei den Strukturelementen c und/oder d C durch Si ersetzt sein kann, oder
- wahlweise a oder b oder e oder f NR²² ist, mit R²² = Alkyl mit C₁-C₁₀, Cycloalkyl mit C₃-C₂₀, Alkenyl mit C₂-C₂₀, Alkinyl mit C₂-C₂₀, Aryl, Heteroaryl, oder
- wahlweise a und f oder b und e NR sind, mit R²² = Alkyl mit C₁-C₂₀, Cycloalkyl mit C₃-C₂₀, Alkenyl mit C₂-C₂₀, Alkinyl mit C₂-C₂₀, Aryl, Heteroaryl,
- wobei die Bindungen a-c, b-d, c-e und d-f, aber nicht gleichzeitig a-c und c-e und nicht gleichzeitig b-d und d-f, ungesättigt sein können,
- wobei die Bindungen a-c, b-d, c-e und d-f Teil eines gesättigten oder ungesättigten Ringsystems sein können, welches auch die Heteroelemente O, S, Se, N, P, Se, Ge, Sn enthalten kann, oder
- die Bindungen a-c, b-d, c-e und d-f Teil eines aromatischen oder kondensierten aromatischen Ringsystems sind, welches auch die Heteroelemente O, S, Si, N enthalten kann,
- wobei das Atom E ein Hauptgruppenelement ist, bevorzugt ausgewählt aus der Gruppe C, N, P, As, Sb,
- wobei das Strukturelement a-e-b wahlweise Bestandteil eines gesättigten oder ungesättigten Ringsystems ist, welches auch die Heteroelemente O, S, Se, N, P, Si, Ge, Sn enthalten kann, oder
- das Strukturelement a-E-b wahlweise Bestandteil eines aromatischen Ringsystems ist, welches auch die Heteroelemente O, S, Se, N enthalten kann.

10. Halbleitermaterial nach Anspruch 9, **dadurch gekennzeichnet, daß** der Dotand die folgender Struktur **Ia** aufweist:

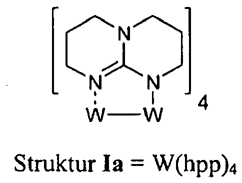

11. Halbleitermaterial nach einem der Ansprüche 5 bis 7, **dadurch gekennzeichnet, daß** der Dotand ein Alkali- und/oder Erdalkalimetall ist.

12. Halbleitermaterial nach einem der vorangehenden Ansprüche 5 bis 11, **dadurch gekennzeichnet, daß** das Matrixmaterial ein Energieniveau für das unterste unbesetzte Molekülorbital (LUMO) aufweist, welches sich um 0-0,5 V gegenüber dem lonisationspotential (HOMO) des Dotanden, unterscheidet.

13. Halbmaterial nach einem der Ansprüche 5 bis 12, **dadurch gekennzeichnet, daß** das Matrixmaterial ein LUMO-Energieniveau aufweist, welches tiefer liegt als das Ionisationspotential (HOMO) des Dotanden.

14. Halbleitermaterial nach einem der vorangehenden Ansprüche 5 bis 13, **dadurch gekennzeichnet, daß** die Konzentration des Dotanden 0,5 bis 25 Masseprozent beträgt.

15. Halbleitermaterial nach einem der vorangehenden Ansprüche 5 bis 14, **dadurch gekennzeichnet, daß** die Glasübergangstemperatur Tg des Matrixmaterials höher ist als diejenige von 4,7-Diphenyl-1,10-phenantrolin (Bphen).

16. Halbleitermaterial nach einem der vorangehenden Ansprüche 5 bis 15, **dadurch gekennzeichnet, daß** das Matrixmaterial eine Verdampfungstemperatur von mindestens 200°C aufweist.

17. Organische Leuchtdiode, welche ein Halbleitermaterial nach einem der vorangehenden Ansprüche 5 bis 16 umfaßt.

18. 5,6-Dihydrophenanthrolin gemäß der Struktur **7**: 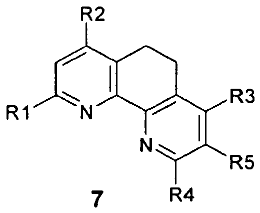 wobei R¹, R², R³, R⁴ und R⁵ wie in Anspruch 1 definiert sind.

## Claims

1. Asymmetrically substituted phenanthrolines with the following structure **8**. 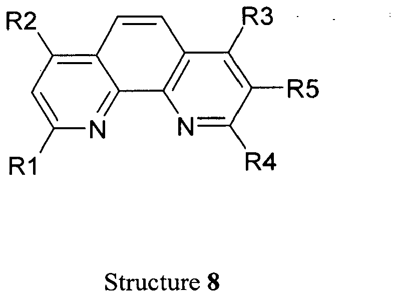 in which:
R¹ and R² are independently selected from aryl and heteroaryl;
R³ is aryl or heteroaryl;
R⁴ is aryl, heteroaryl, alkyl with C₁-C₂₀ or cycloalkyl with C₃-C₂₀;
R⁵ is CN, COOalkyl, COaryl or COalkyl.

2. A method for the production of asymmetrically substituted phenanthrolines comprising the following steps:
(ia) conversion of a substituted methylene hydroquinolinone **5** with enamine
under basic conditions for the preparation of a 1,4,5,6-tetrahydrophenanthroline **6** und subsequent oxidation for the preparation of a 5,6-dihydrophenanthroline **7** according to the following reaction scheme: 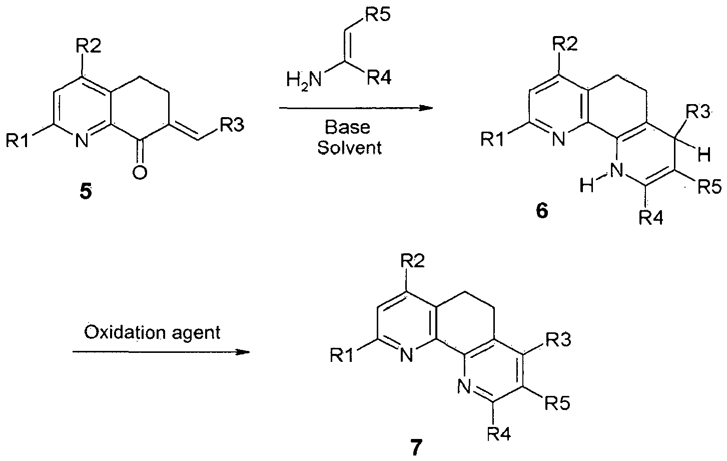
(iia) aromatization of the 5,6-dihydrophenanthroline **7** to the asymmetrically
substituted phenanthroline **8** according to the following reaction scheme: 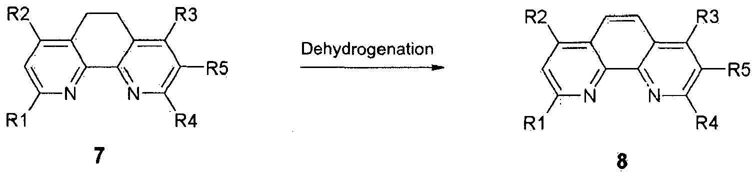 or
(ib) conversion of a substituted methylene hydroquinolinone **5** with enamine
under basic conditions in order to prepare a 1,4,5,6-tetrahydrophenanthroline **6** and subsequent direct oxidation to the asymmetrically substituted phenanthroline **8**,
in which R¹, R², R³, R⁴ and R⁵ are defined as above in Claim 1.

3. The method according to Claim 2, **characterized in that** methane sulfonic acid or chloranil is used for the oxidation of the 1,4,5,6-tetrahydrophenanthroline **6** in step (i).

4. The method according to Claim 2 or 3, **characterized in that** the aromatization takes place in step (ii) by Pd-catalyzed dehydrogenation by Pd/C.

5. Doped organic semiconductor material comprising at least one organic matrix material doped with at least one doping agent, which matrix material is an asymmetrically substituted phenanthroline **8** in accordance with Claim 1.

6. The semiconductor material according to Claim 5, **characterized in that** the matrix material is reversibly reducible.

7. The semiconductor material according to Claim 5, **characterized in that** the matrix material decomposes in a reduction into stable, redox-reactive components.

8. The semiconductor material according to any one of Claims, 5 to 7, **characterized in that** the doping agent is a metallic complex.

9. The semiconductor material according to Claim 8, **characterized in that** the metallic
complex has a structure I: 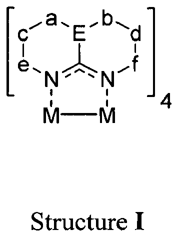 in which M is a transition metal, preferably Mo or W; and in which
- the structural elements a-f can have the significance: a= -CR₉R₁₀-, b = -CR₁₁R₁₂-, c = -CR₁₃R₁₄-, d = R₁₅R₁₆-, e = -CR₁₇R₁₈- and f = -CR₁₉R₂₀-, in which R₉-R₂₀ are independently hydrogen, alkyl with C₁-C₂₀, cycloalkyl with C₃-C₂₀, alkenyl with C₁-C₂₀, alkynyl with C₂-C₂₀, aryl, heteroaryl, -NR²¹R²¹ or -OR²¹, in which R²¹ = alkyl with C₁-C₂₀, cycloalkyl with C₃-C₂₀, alkenyl with C₂-C₂₀, alkynyl with C₂-C₂₀, aryl or heteroaryl, in which preferably R₉, R₁₁, R₁₃, R₁₅, R₁₇, R₁₉ = H and R₁₀, R₁₂, R₁₄, R₁₆, R₁₈, R₂₀ = alkyl with C₁-C₂₀, cycloalkyl with C₃-C₂₀, alkenyl with C₂-C₂₀, alkynyl with C₂-C₂₀, aryl, heteroaryl, -NR²¹R²¹ or -OR²¹, or
- in the structural elements c and/or d C can be replaced by Si, or
- a or b or e or f is selectively NR²², with R²² = alkyl with C₁-C₂₀, cycloalkyl with C₃-C₂₀, alkenyl with C₂-C₂₀, alkynyl with C₂-C₂₀, aryl, heteroaryl, or
- a and f or b and e are selectively NR, with R²² = alkyl with C₁-C₂₀, cycloalkyl with C₃-C₂₀, alkenyl with C₂-C₂₀, alkynyl with C₂-C₂₀, aryl, heteroaryl,
- in which the bonds a-c, b-d, c-e and d-f, but not simultaneously a-c and c-e, and not simultaneously b-d and d-f, can be unsaturated,
- in which the bonds a-c, b-d, c-e and d-f can be part of a saturated or unsaturated ring system that can also contain the heteroelements O, S, Se, N, P, Se, Ge, Sn, or
- the bonds a-c, b-d, c-e and d-f are part of an aromatic or condensed aromatic ring system that can also contain the heteroelements O, S, Si, N,
- in which the atom E is a main group element preferably selected from the group of C, N, P, As, Sb,
- in which the structural element a-e-b is selectively component of a saturated or unsaturated ring system that can also contain the heteroelements O, S, Se, N, P, Si, Ge, Sn, or
- the structural element a-E-b is selectively component of an aromatic ring system that can also contain the heteroelements O, S, Se, N.

10. The semiconductor material according to Claim 9, **characterized in that** the doping agent has the following structure **Ia**:

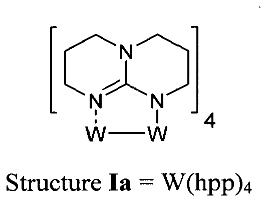

11. The semiconductor material according to any one of Claims 5 to 7, **characterized in that** the doping agent is an alkali- and/or alkaline-earth metal.

12. The semiconductor material according to any one of the preceding Claims 5 to 11, **characterized in that** the matrix material has an energy level for the lowest unoccupied molecular orbital (LUMO) that differs by 0-0.5 V from the ionization potential (HOMO) of the doping agent.

13. The semiconductor material according to any one of Claims 5 to 12, **characterized in that** the matrix material has a LUMO energy level that is lower than the ionization potential (HOMO) of the doping agent.

14. The semiconductor material according to any one of the preceding Claims 5 to 13, **characterized in that** the concentration of the doping agent is 0.5 to 25 % by weight.

15. The semiconductor material according to any one of the preceding Claims 5 to 14, **characterized in that** the glass transition point Tg of the matrix material is higher than that of 4,7-diphenyl-1,10-phenanthroline (Bphen).

16. The semiconductor material according to any one of the preceding Claims 5 to 15, **characterized in that** the matrix material has an evaporation temperature of at least 200°C.

17. An organic light-emitting diode comprising a semiconductor material according to any one of the preceding Claims 5 to 16.

18. 5,6-Dihydrophenanthroline according to the structure **7**: 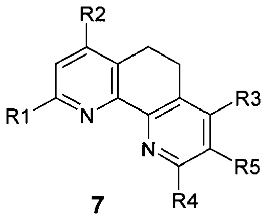 in which R¹, R², R³, R⁴ and R⁵ are defined as in Claim 1.

## Revendications

1. Phénanthrolines asymétriquement substituées répondant à la structure **8** suivante : 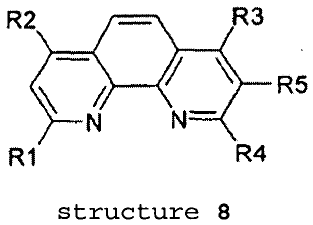 dans laquelle :
R¹ et R² sont choisis de façon indépendante parmi aryle et
hétéroaryle ;
R³ représente aryle ou hétéroaryle ;
R⁴ représente aryle, hétéroaryle, alkyle en C₁-C₂₀ ou
cycloalkyle en C₃-C₂₀ ;
R⁵ représente CN, COO-alkyle, CO-aryle ou CO-alkyle.

2. Procédé de préparation de phénanthrolines asymétriquement substituées, comprenant les étapes suivantes consistant à :
(ia) faire réagir une méthylène-hydroquinolinone substituée **5** avec une énamine dans des conditions basiques, pour obtenir une 1,4,5,6-tétrahydrophénanthroline **6** laquelle est ensuite oxydée pour obtenir une 5,6-dihydrophénanthroline **7** selon le schéma de réaction suivant : 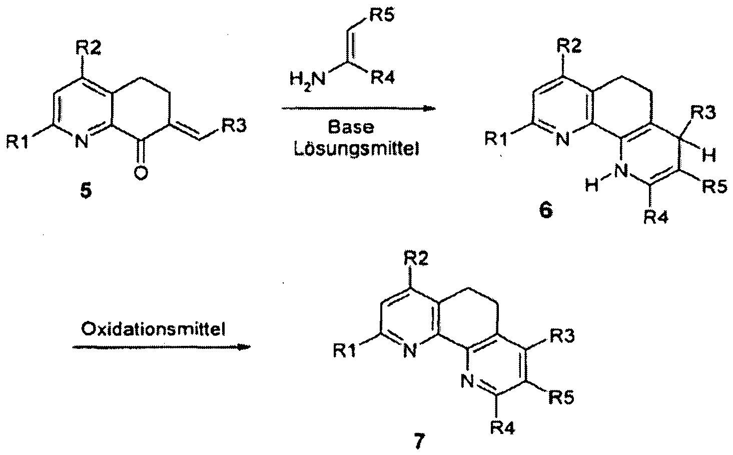
| Lôsungsmittel | solvant |
|---|---|
| Oxidationsmittel | agent d'oxydation |
(iia) transformer la 5,6-dihydrophénanthroline **7** en
composé aromatique, pour obtenir la phénanthroline asymétriquement substituée **8**, selon le schéma de réaction suivant : 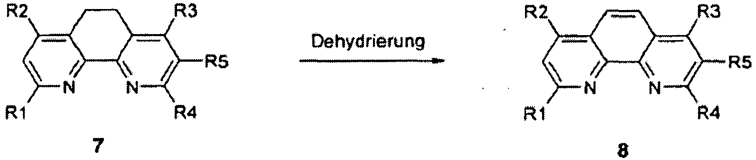
| | |
|---|---|
| Dehydrierung | déshydrogénation |
ou
(ib) faire réagir une méthylène-hydroquinolinone substituée **5** avec une énamine dans des conditions basiques, pour obtenir une 1,4,5,6-tétrahydrophénanthroline **6** laquelle est ensuite oxydée de manière à obtenir directement la phénanthroline asymétriquement substituée **8**,
dans lequel R¹, R², R³, R⁴ et R⁵ correspondent aux définitions indiquées ci-dessus dans la revendication 1.

3. Procédé selon la revendication 2, **caractérisé en ce qu'**à l'étape (i), on met en oeuvre de l'acide méthanesulfonique ou du chloranile pour oxyder le 1,4,5,6-tétrahydrophénanthroline **6**.

4. Procédé selon les revendications 2 ou 3, **caractérisé en ce qu'**à l'étape (ii), la transformation en composé aromatique est réalisée en mettant en oeuvre une déshydrogénation Pd-catalysée par Pd/C.

5. Matériau semi-conducteur organique dopé, comprenant au moins un matériau de matrice organique qui est dopé au moyen d'au moins un dopant, ledit matériau de matrice étant une phénanthroline asymétriquement substituée **8** selon la revendication 1.

6. Matériau semi-conducteur selon la revendication 5, **caractérisé en ce que** le matériau de matrice peut subir une réduction réversible.

7. Matériau semi-conducteur selon la revendication 5, **caractérisé en ce que** lors d'une réduction, le matériau de matrice se décompose en composants susceptibles de subir une réaction d'oxydoréduction.

8. Matériau semi-conducteur selon l'une des revendications 5 à 7, **caractérisé en ce que** ledit dopant est un complexe métallique.

9. Matériau semi-conducteur selon la revendication 8, **caractérisé en ce que** ledit complexe métallique présente une structure **I** : 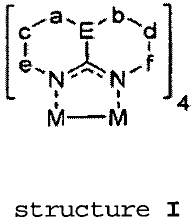 M étant un métal de transition, de préférence Mo ou W ; et
- les éléments structurels a-f pouvant avoir les significations suivantes : a = -CR₉R₁₀-, b = -CR₁₁R₁₂-, c = -CR₁₃R₁₄-, d = R₁₅R₁₆-, e = -CR₁₇R₁₈- et f = -CR₁₉R₂₀-, R₉ à R₂₀ représentant de façon indépendante hydrogène, alkyle en C₁-C₂₀, cycloalkyle en C₃-C₂₀, alcényle en C₁-C₂₀, alcynyle en C₂-C₂₀, aryle, hétéroaryle, -NR²¹R²¹ ou - OR²¹, dans lesquels R²¹ représente alkyle en C₁-C₂₀, cycloalkyle en C₃-C₂₀, alcényle en C₂-C₂₀, alcynyle en C₂-C₂₀, aryle ou hétéroaryle, R₉, R₁₁, R₁₃, R₁₅, R₁₇, R₁₉ étant préférentiellement égaux à H alors que R₁₀, R₁₂, R₁₄, R₁₆, R₁₈, R₂₀ représentent préférentiellement alkyle en C₁-C₂₀, cycloalkyle en C₃-C₂₀, alcényle en C₂-C₂₀, alcynyle en C₂-C₂₀, aryle, hétéroaryle, -NR²¹R²¹ ou -OR²¹, ou
- C pouvant être remplacé par Si chez les éléments structurels c et/ou d, ou
- a ou b ou e ou f pouvant, sélectivement, représenter NR²², dans lequel R²² représente alkyle en C₁-C₂₀, cycloalkyle en C₃-C₂₀, alcényle en C₂-C₂₀, alcynyle en C₂-C₂₀, aryle, hétéroaryle, ou
- a et f ou bien b et e pouvant, sélectivement, représenter NR, dans lequel R²² représente alkyle en C₁-C₂₀, cycloalkyle en C₃-C₂₀, alcényle en C₂-C₂₀, alcynyle en C₂-C₂₀, aryle, hétéroaryle,
- les liaisons a-c, b-d, c-e et d-f pouvant être insaturées, à condition que a-c et c-c ne le soient jamais simultanément et que b-d et d-f ne le soient jamais simultanément,
- les liaisons a-c, b-d, c-e et d-f pouvant faire partie d'un système cyclique saturé ou insaturé lequel peut également contenir les hétéroéléments O, S, Se, N, P, Se, Ge, Sn, ou
- les liaisons a-c, b-d, c-e et d-f faisant partie d'un système cyclique aromatique ou condensé lequel peut également contenir les hétéroéléments O, S, Si, N.
- l'atome E étant un élément de groupe principal qui est préférentiellement choisi dans le groupe de C, N, P, As, Sb,
- l'élément structurel a-e-b faisant optionnellement partie d'un système cyclique saturé ou insaturé lequel peut également contenir les hétéroéléments O, S, Se, N, P, Si, Ge, Sn, ou
- l'élément structurel a-E-b faisant optionnellement partie d'un système cyclique aromatique lequel peut également contenir les hétéroéléments O, S, Se, N.

10. Matériau semi-conducteur selon la revendication 9, **caractérisé en ce que** ledit dopant présente la structure **Ia** suivante :

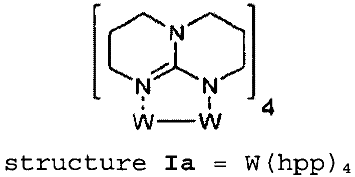

11. Matériau semi-conducteur selon l'une des revendications 5 à 7, **caractérisé en ce que** ledit dopant est un métal alcalin et/ou alcalino-terreux.

12. Matériau semi-conducteur selon l'une des revendications 5 à 11 précédentes, **caractérisé en ce que**, chez le matériau de matrice, l'orbitale moléculaire basse vacante (LUMO) présente un niveau d'énergie sui est différent de 0 à 0,5 V par rapport au potentiel d'ionisation (HOMO) du dopant.

13. Demi-matériau selon l'une des revendications 5 à 12, **caractérisé en ce que** le matériau de matrice présente un niveau d'énergie LUMO qui est inférieur au potentiel d'ionisation (HOMO) du dopant.

14. Matériau semi-conducteur selon l'une des revendications 5 à 13 précédentes, **caractérisé en ce que** la concentration du dopant est comprise entre 0,5 et 25 % en masse.

15. Matériau semi-conducteur selon l'une des revendications précédentes 5 à 14, **caractérisé en ce que** la température de transition vitreuse Tg du matériau de matrice est supérieure à celle de 4,7-diphényl-1,10-phénantroline (Bphen).

16. Matériau semi-conducteur selon l'une des revendications 5 à 15 précédentes, **caractérisé en ce que** le matériau de matrice présente une température évaporation d'au moins 200 °C.

17. Diode électroluminescente organique, comprenant un matériau semi-conducteur selon l'une des revendications 5 à 16 précédentes.

18. 5,6-Dihydrophénanthroline répondant à la structure 7 : 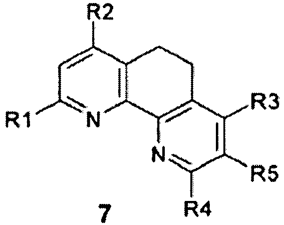 dans laquelle R¹, R², R³, R⁴ et R⁵ sont définis comme dans la revendication 1.
